Europäisches Patentamt

European Patent Office

Office européen des brevets

(11) Publication number: **0 338 404**
**A1**

# EUROPEAN PATENT APPLICATION

(21) Application number: **89106517.9**

(51) Int. Cl.⁴: **A61K 31/57**

(22) Date of filing: **12.04.89**

(30) Priority: **12.04.88 US 180407**

(43) Date of publication of application:
**25.10.89 Bulletin 89/43**

(84) Designated Contracting States: .
**AT BE CH DE ES FR GB GR IT LI LU NL SE**

(71) Applicant: **Bristol-Myers Company**
**345 Park Avenue**
**New York New York 10154(US)**

(72) Inventor: **Markison, Brian A.**
**635 Kirkwood Drive**
**Evansville Indiana 47715(US)**

(74) Representative: **Kinzebach, Werner, Dr. et al**
**Patentanwälte Reitstötter, Kinzebach und**
**Partner Sternwartstrasse 4 Postfach 86 06 49**
**D-8000 München 86(DE)**

(54) **The use of megestrol acetate for preparing a pharmaceutical composition for treating AIDS cachexia.**

(57) Treatment with megestrol acetate will retard and/or reduce cachexia associated with acquired immunodeficiency syndrome.

EP 0 338 404 A1

# THE USE OF MEGESTROL ACETATE FOR PREPARING A PHARMACEUTICAL COMPOSITION FOR TREATING AIDS CACHEXIA

## Background of the Invention

### 1. Field of the Invention

This invention relates to the use of megestrol acetate for preparing a pharmaceutical composition for retarding and/or reducing cachexia associated with aquired immunodeficiency syndrome in humans.

### 2. Description of the Prior Art

The acquired immunodeficiency syndrome (AIDS) was first recognized as a distinct clinical entity in 1981 when the Center for Disease Control received multiple reports of two rare diseases, Pneumocystis Carinii pneumonia and Kaposi's Sarcoma, in previously healthy men in both New York and California. The disease was characterized initially as being associated with eleven different infections and diseases including the above-mentioned Pneumocystis Carinii pneumonia and Kaposi's Sarcoma. After HIV was discovered to be the etiologic agent in AIDS, however, and sensitive and specific HIV antibody tests became available, the manifestations of HIV infection have become better recognized and defined. It is now apparent that some progressive, seriously disabling and even fatal conditions which are non-infectious and non-malignant in nature affect a substantial number of HIV infected persons.

The most recent revision of the surveillance definition of AIDS by the Center for Disease Control takes some of these newly recognized progressive conditions into consideration. The new definition includes such conditions as HIV dementia and the HIV wasting syndrome. The HIV wasting syndrome has been defined as a profound involuntary weight loss that is greater than ten percent of premorbid weight associated with either chronic diarrhea (lasting more than one month), weakness or documented fevers and which occurs in the absence of recognized causes other than the HIV infection.

The exact prevalence of weight loss associated with AIDS is unknown but thought by many to be quite common. The new definition of AIDS requires reporting of significant weight loss and, as a result, extensive prevalence data may become available in the near future. Evidence from small studies carried out to date, however, indicates that a large proportion of AIDS patients have a >10% weight loss from their premorbid weight. Such weight loss and commonly associated anorexia and weakness (described hereinafter as "AIDS cachexia") have serious physical and mental consequences to AIDS patients which heretofore have not been addressed.

Although the cachexia associated with AIDS is often severe and debilitating, there is almost no information available regarding the mechanism of AIDS cachexia. As with patients with advanced cancer, it is likely to be multifactorial in etiology, although the mechanism of cachexia in patients with AIDS is unlikely to be the same as that seen in patients with cancer cachexia.

Megestrol acetate is presently marketed as an oral antineoplastic agent for the treatment of carcinoma of the breast or endometrium. Oral dosages in the range of about 40-800 mg/day are indicated for these tumors (see U.S. Patent 4,370,321). One of the side effects seen with megestrol acetate therapy has been weight gain.

Recently, the administration of higher oral doses of megestrol acetate up to 1600 mg/day in the treatment of breast cancer has been reported (see Semin. Hematol. 24(2), pg. 48-66, April 1987). With the higher doses of the compound a significant weight gain was seen along with increased appetite and an increased sense of well-being. Such results led the investigators to suggest that high dose megestrol acetate may be effective in the treatment of cancer cachexia (see J. Am. Med. Assoc. 257(9), pg. 1195-1198, 1987).

## Summary of the Invention

This invention is predicated upon the discovery that megestrol acetate when administered either orally or parenterally, but preferably orally, in effective non-toxic amounts to humans affected by cachexia associated with acquired immunodeficiency syndrome will retard and/or reduce cachexia in such patients, even in the absence of other improvements in the underlying disease.

## Detailed Description of the Invention

The dosages of megestrol acetate employed in the present invention can vary from about 40 to 1600 mg/day. Oral administration with three to four divided doses is preferred. The megestrol acetate may be administered by itself or in combination with other agents useful in suppressing the HIV virus, e.g. 3'-azido-3'-deoxythymidine (AZT). The megestrol acetate may be given in standard pharmaceutical formulations such as those already used in the treatment of cancer. Other suitable pharmaceutical formulations may be readily prepared by those skilled in the art. If adequate response is observed the patient is maintained on the megestrol acetate as long as there is evidence of cachexia retardation and/or reduction.

Patients testing HIV-positive were treated with oral doses of megestrol acetate, 80 mg. four times a day. These patients were all at very different points in the clinical course of their disease. All, however, were on AZT and were seen on a weekly basis. Three of the patients had no known gastrointestinal disease. None of the patients had exhibited any diarrhea over the past three months. All of the patients had lost greater than 10% of their pre-illness body weight. All were treated with megestrol acetate for between four and six weeks.

Patient No. 1 is a white bisexual male who was diagnosed with AIDS on the basis of Kaposi's Sarcoma with known gastrointestinal involvement (asymptomatic).· Shortly before entering the investigation he was found to have a positive blood culture for cyclomegalovirus. He also had a mild neurologic dysfunction for the previous few months. His baseline weight before illness was 200 pounds and at the time of initiation of megestrol acetate therapy he weighed 141 pounds. After one week of therapy he gained one pound. On questioning it was discovered that he had decreased his dose and was taking megestrol acetate only three times a day because "he woke up in the middle of the night too hungry". He gained another pound in the second week and then at week three his weight stabilized. With further questioning it was discovered that when his AZT has been discontinued the previous week because of granulocytopenia, he had discontinued all of his medications. Upon restarting megestrol acetate therapy his weight again increased at week four for a total weight gain over three weeks of therapy of 5 pounds. He remained on therapy for eleven weeks and gained 19 pounds. At that point he had significant deterioration in his clinical status secondary to progressive Kaposi's Sarcoma and neurologic dysfunction and he chose to discontinue therapy.

Patient No. 2 is a homosexual black male who did not carry a diagnosis of AIDS but was on AZT because of a helper T cell number below 200. Prior to his illness he weighed 125 pounds and, at the initiation of megestrol acetate therapy, he weighed 111 pounds. He had no side effects from the therapy and reported a marked improvement in his appetite with a weight gain of 21 pounds over a 16-week therapy period. He has discontinued therapy because he is now weighing more than his pre-illness weight and "feels fat".

Patient No. 3 was a homosexual white male who was given a diagnosis of AIDS on the basis of multiple episodes of Pneumocystis Carinii pneumonia and Kaposi's Sarcoma involving his oral mucosa. His pre-illness weight was 185 pounds and, at the initiation of megestrol acetate therapy, he weighed 161.5 pounds. The first week of therapy he gained 1-1/2 pounds, subsequently lost 2 pounds, and then steadily gained weight so that over a 6-week period he gained 6-1/2 pounds. On questioning him regarding side effects his only complaint was that his appetite was increased to such an extent that he "felt full all the time". The patient discontinued therapy when he was hospitalized for a septic episode associated with neurologic dysfunction and fairly rapid deterioration in his overall clinical status. He expired one month after discontinuation of the drug.

Patient No. 4 was a white homosexual male who carried a diagnosis of AIDS because of multiple episodes of Pneumocystis Carinii pneumonia. His weight prior to illness was 145 pounds and, at the initiation of megestrol acetate therapy, he weighed 120 pounds. He was no longer receiving AZT when megestrol acetate therapy was initiated. He gained weight quickly and, over a 10-week period, he gained fourteen pounds. Therapy was discontinued when the patient developed nausea, vomiting and dehydration. He developed Pneumocystis Carinii pneumonia and expired two and one-half weeks after discontinuation of megestrol acetate.

Patient No. 5 is a homosexual white male who carries a diagnosis of AIDS on the basis of multiple episodes of Pneumocystis Carinii pneumonia. Prior to his illness, his normal body weight was 135 pounds. At the initiation of megestrol acetate therapy he weighed 101 pounds and had been on Retrovir for eight months. Although his weight gain was slow, after nine week of therapy he had gained nine pounds. At that time, he developed recurrent Pneumocystis Carinii pneumonia with significant nausea and vomiting associated with the necessary therapy. He was unable to tolerate oral medications at that time and the megestrol acetate was discontinued. The patient has recovered very slow from this most recent episode of Pneumocystis Carinii pneumonia. Over the month of his illness he has lost twelve pounds and is presently weighing 98 pounds off therapy. He has not yet made a decision as to whether the megestrol acetate will be re-started.

Patient No. 6 is a bisexual white male who carries a diagnosis of AIDS on the basis of a single episode of Pneumocystis Carinii pneumonia. His pre-illness body weight was 165 pounds and at the time of initiation of megestrol acetate he weighed 150 pounds. He had been on AZT for a few months at the time of initiation of megestrol acetate therapy. Over a sixteen-week period, the patient has gained twenty-four pounds and is now greater than his initial body weight. Having surpassed his pre-illness body weight, he has decreased the dose to one 40 mg. tablet, four times a day, and continues to note a good appetite and an overall improved sense of well-being.

Patient No. 7 is a white homosexual male who carries a diagnosis of AIDS because of multiple opportunistic infections and Kaposi's sarcoma. His premorbid weight was 155 pounds, and at the time of initiation of megestrol acetate therapy he weighed 129 pounds. He was initially started on AZT five months prior to initiation of megestrol acetate. He was, however, unable to tolerate AZT because of bone marrow suppression, and he was no longer receiving the drug at the time megestrol acetate therapy was initiated. He gained 17 pounds over a very short period of five weeks and then developed pancytopenia and rapid deterioration in his clinical status. He was unable to tolerate oral medications and expired approximately one month after discontinuing his megestrol acetate therapy.

Patient No. 8 is a white homosexual male who carried a diagnosis of AIDS on the basis of an opportunistic infection and Pneumocystis Carinii pneumonia. His pre-illness weight was 145 pounds., and at the initiation of megestrol acetate therapy he weighed 125 pounds. He was treated for only two weeks, with a weight gain of two pounds, when he developed a progressive dementia which required institutionalization and discontinuation of therapy. The patient expired approximately one month later.

Patient No. 9 is a white homosexual male who carries a diagnosis of AIDS on the basis of multiple episodes of Pneumocystis Carinii pneumonia. His pre-illness weight was 140 pounds., and at the time of initiation of therapy he weighed 120 pounds. Over ten weeks of therapy he has gained 8-1/2 pounds and does note a general improvement in his sense of well-being and appetite stimulation. He is continuing on therapy without side effects.

Patient No. 10 is a bisexual white male who carries a diagnosis of AIDS based on an episode of Pneumocystis Carinii pneumonia, as well as other opportunistic infections. His pre-illness body weight was 164 pounds, and at the time of megestrol acetate therapy he weighed 131 pounds. His AZT was started a few weeks after the initiation of megestrol acetate. Over seven weeks of therapy he has gained 9 pounds and notes a marked improvement in his overall sense of well-being.

Six patients reported a general improvement in their overall sense of well being. All patients noted a general improvement in their appetite. There were no significant side effects noted and no edema was found during weekly physical examinations.

## Claims

1. The use of megestrol acetate for preparing a pharmaceutical composition for retarding and/or reducing cachexia associated with acquired immune deficiency syndrome.

2. The use according to claim 1 wherein the pharmaceutical composition is in a form suitable for oral dosage.

3. The use according to claim 1 or 2 wherein the dosage of megestrol acetate is from about 40 to about 1600 mg/day.

4. The use according to claim 3 wherein megestrol acetate is administered in divided doses.

# DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,D | JOURNAL OF AMERICAN MEDICAL ASSOCIATION, vol. 257, no. 9, 6th March 1987, pages 1195-1198; N.S. TCHEKMEDYIAN et al.: "High-dose megestrol acetate. A possible treatment for cachexia" * The whole article * --- | 1-4 | A 61 K 31/57 |
| X | SEMIN. ONCOL., vol. 13, no. 4, supplement 4, December 1986, pages 37-43; N.S. TCHEKMEDYIAN et al.: "Appetite stimulation with megestrol acetate in cachectic cancer patients" * The whole article * --- | 1-4 | |
| X | PROCEEDINGS OF AACR, AMERICAN ASSOCIATION CANCER RESEARCH, vol. 27, no. 209, March 1986, no. 829; N.S. TCHEKMEDYIAN et al.: "Weight gain with high dose (HD) megestrol acetate (MA): A possible treatment for cachexia" * The whole article * --- | 1-4 | TECHNICAL FIELDS SEARCHED (Int. Cl.4) |
| P,X | ANNALS OF INTERNAL MEDICINE, vol. 109, no. 10, 15th November 1988, pages 840-841, American College of Physicians; J.H. VON ROENN et al.: "Megestrol acetate for treatment of cachexia associated with human immunodeficiency virus (HIV) infection" * The whole article * --- | 1-4 | A 61 K |
| Y | EP-A-0 222 385 (RESEARCH DEVELOPMENT CORPORATION OF JAPAN) * Page 2, lines 1-2; page 3, lines 28-40 * --- | 1-4 | |
| | -/- | | |

The present search report has been drawn up for all claims

| Place of search | Date of completion of the search | Examiner |
|---|---|---|
| THE HAGUE | 11-08-1989 | GAC G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................................................

& : member of the same patent family, corresponding document

EPO FORM 1503 03.82 (P0401)

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,P | CLINICAL PHARMACY, vol. 7, July 1988, pages 514-526, American Society of Hospital Pharmacists; C.J. WORDELL et al.: "Treatment of Pneumocystis carinii pneumonia in patients with AIDS" * The whole article * | 1-4 | |

TECHNICAL FIELDS SEARCHED (Int. Cl.4)

The present search report has been drawn up for all claims

| Place of search | Date of completion of the search | Examiner |
|---|---|---|
| THE HAGUE | 11-08-1989 | GAC G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)